# EUROPEAN PATENT APPLICATION

(11) **EP 2 772 247 A1**
(43) Date of publication of application: **03.09.2014**
(21) Application number: 11876753.2
(22) Date of filing: 01.12.2011
(51) Int. Cl.: A61K 8/89, A61K 47/30, A61K 9/16, A61K 8/02, A61Q 17/04, A61Q 1/00

(54) **SURFACE-MODIFIED ULTRAVIOLET BLOCKING POWDER AND ULTRAVIOLET BLOCKING COMPOSITION INCLUDING SAME**

(71) Applicant: KCI Co.,ltd, Seoul 153-803 (KR)
(72) Inventor: YU, Won Woo, Seoul 151-803 (KR); PARK, Jung Ho, Yongin-si Gyeonggi-do 446-567 (KR)
(74) Representative: Von Rohr Patentanwälte Partnerschaft mbB
(86) International application number: PCT/KR2011/009272
(87) International publication number: WO 2013/081217

(57) **Abstract**

The present invention relates to a surface-modified ultraviolet blocking powder obtained by reacting an ultraviolet blocking powder including a chloroacetyl group, a phospholipid monomer including an unsaturated bonding, and a catalyst inducing the reaction of the chloroacetyl group and the unsaturated bonding. According to the surface-modified ultraviolet blocking powder of the present invention, the surface may be modified using an effective method, and through chemical bonding (covalent bonding) and as a result of surface modification, the stability of the powder is high. In addition, since the surface modification is performed by using a biocompatible component, the ultraviolet blocking composition including the surface-modified ultraviolet blocking powder may provide ultraviolet blocking properties and biocompatibility at the same time.

## Description

### Technical Field

The present invention relates to a surface-modified ultraviolet blocking powder and an ultraviolet blocking composition comprising the same.

### Background Art

Typically useful for protecting the skin from ultraviolet (UV) rays, sunscreens are classified into (i) inorganic sunscreens such as titanium dioxide and zinc oxide, and (ii) organic sunscreens, such as ethylhexyl methoxycinnamate, butylmethoxydibenzoylmethane, ethylhexyl triazone, glyceryl PABA, drometrizole, benzophenone-3, benzophenone-4, benzophenone-8, cinoxate, octocrylene, ethylhexyl dimethyl PABA, 2-phenylbenzimidazole-5-sulfonic acid, ethylhexyl salicylate and homosalate.

Among these, titanium dioxide, which functions to effectively block UVB, has been widely used in sun care products serving as a makeup base. Inorganic sunscreens include metal oxides such as titanium dioxide, zinc oxide and cerium oxide, and are broadly useful because of their excellent UV blocking capability and safety. As well, titanium dioxide is cheap and has high covering capability and is thus diversely utilized in sun protection products and also makeup cosmetics.

However, inorganic sunscreens such as titanium dioxide have defects such as high photo-activity that leads to skin irritation, a whitening phenomenon when applied on the skin, a decrease in the titer due to the formation of a complex upon mixing with a predetermined organic UV material, and a rough feeling due to its tetrahedral morphology.

To solve the above problems, ongoing is research and development into minimizing the amount of titanium dioxide particles required for desired UV blocking effectiveness (US Patent No. 6,123,927), preparation of a composite comprising talc coated with titanium dioxide, preparation of a powder by simply mixing an inorganic powder with titanium dioxide or an organic resin with titanium dioxide, incorporation of titanium alkoxide on or in a substrate through hydrolysis using a solgel process, and incorporation of titanium dioxide. Furthermore, methods of incorporating formed titanium dioxide particles in a resin and hybridizing them (Japanese Patent Application Publication No. 2001-00280147) are under study, but the prior methods suffer from instability due to the simple mixing or the simple coating forms and make it difficult to enhance biocompatibility.

Meanwhile, a phosphorylcholine analogous group-containing polymer is based on a phospholipid analogous structure derived from biomembranes, and is known to be superior in biocompatibility related with inactivation of blood components or non-adsorption of biomaterials, antifouling properties and moisture retention. Furthermore, synthesis of the phosphorylcholine analogous group-containing polymer for development of biomaterials including functions thereof, and the end use thereof, are being actively researched and developed. The phosphorylcholine analogous group-containing polymer includes a phosphorylcholine analogous group-containing monomer. Especially, 2-(methacryloyloxy)ethyl-2'-(trimethylammonio)ethylphosphate [which is also referred to as 2-methacryloyloxyethyl phosphorylcholine, and hereinafter abbreviated to MPC] is known to impart outstanding biocompatibility and moisture retention due to the presence of a phosphorylcholine analogous group structurally similar to phosphatidyl choline that is a cell wall component.

### Disclosure

### Technical Problem

Accordingly, the present invention has been made keeping in mind the above problems occurring in the related art, and an object of the present invention is to provide a surface-modified UV blocking powder having UV blocking characteristics and superior biocompatibility.

Another object of the present invention is to provide a surface-modified UV blocking powder, which may be prepared using an effective method.

A further object of the present invention is to provide a UV blocking composition, a coloring composition or a cosmetic composition, containing the surface-modified UV blocking powder.

### Technical Solution

In order to accomplish the above objects, the present invention provides a surface-modified UV blocking powder, obtained by reacting a UV blocking powder having a chloroacetyl group, a phospholipid monomer having an unsaturated bond, and a catalyst for inducing the reaction of the chloroacetyl group and the unsaturated bond.

The UV blocking powder having a chloroacetyl group may be represented by Chemical Formula 1 below. 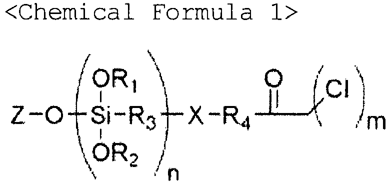 (in Chemical Formula 1, Z is a UV blocking powder, R₁ and R₂ are independently Z, hydrogen or a C1∼C20 aliphatic hydrocarbon with or without a substituent, R₃ is oxygen or a C1∼C20 aliphatic hydrocarbon with or without a substituent and including a carbonyl group, a hydroxyl group, an ether group, an amine group, an amide group or a urethane group in a main chain or at a terminal thereof, R₄ is absent or is a C1∼C20 aliphatic hydrocarbon with or without a substituent and including a carbonyl group, a hydroxyl group, an ether group, an amine group, an amide group or a urethane group in a main chain or at a terminal thereof, X is absent or is -OCONH-, - CONH-, -NH-, -CO-, -O-, -S- or a C1∼C20 aliphatic hydrocarbon with or without a substituent and including a carbonyl group, a hydroxyl group, an ether group, an amine group, an amide group or a urethane group in a main chain or at a terminal thereof, n is 0 or 1, and m is an integer of 1 ∼ 3.)

The UV blocking powder may comprise titanium dioxide, zinc oxide, mica, sericite or talc, and the catalyst may be Mo(CO)₆.

The compound of Chemical Formula 1 may comprise at least one selected from among compounds of the following Chemical Formulas.

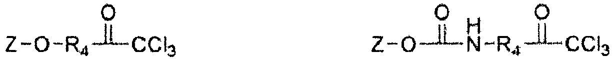

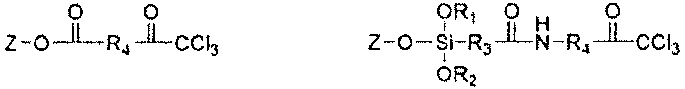

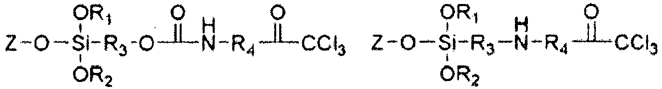

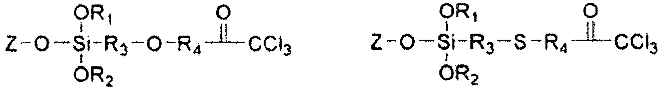

In Chemical Formula 1, when either or both of R₁ and R₂ are Z, all Zs represented in the Chemical Formula may be the same UV blocking powder.

The phospholipid monomer having an unsaturated bond may be a phosphorylcholine analogous group-containing monomer having an unsaturated bond, and may be a phosphorylcholine analogous group-containing monomer represented by Chemical Formula 2 below. 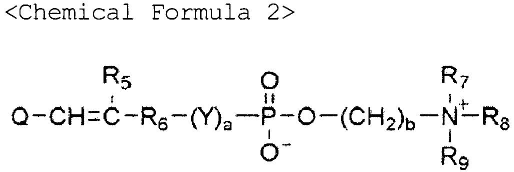 (in Chemical Formula 2, R₆ is a divalent organic residue, Y is a C1∼C6 alkyleneoxy group, Q is a hydrogen atom or R₁₀-O-CO- (wherein R₁₀ is a C1∼C10 alkyl group or a C1∼C10 hydroxyalkyl group), R₅ is a hydrogen atom or a methyl group, R₇, R₈ and R₉ are each independently a hydrogen atom, a C1∼C6 hydrocarbon group or a hydroxyhydrocarbon group, a is 0 or 1, and b is an integer of 2 ∼ 4.)

The phospholipid monomer having an unsaturated bond may comprise at least one selected from among 2-((meth)acryloyloxy)ethyl-2'-(trimethylammonio)ethylphosphate, 3-((meth)acryloyloxy)propyl-2'-(trimethylammonio)ethylphosphate, 4-((meth)acryloyloxy)butyl-2'-(trimethylammonio)ethylphosphate, 5-((meth)acryloyloxy)pentyl-2'-(trimethylammonio)ethylphosphate, 6-((meth)acryloyloxy)hexyl-2'-(trimethylammonio)ethylphosphate, 2-((meth)acryloyloxy)ethyl-2'-(triethylammonio)ethylphosphate, 2-((meth)acryloyloxy)ethyl-2'-(tripropylammonio)ethylphosphate, 2-((meth)acryloyloxy)ethyl-2'-(tributylammonio)ethylphosphate, 2-((meth)acryloyloxy)propyl-2'-(trimethylammonio)ethylphosphate, 2-((meth)acryloyloxy)butyl-2'-(trimethylammonio)ethylphosphate, 2-((meth)acryloyloxy)pentyl-2'-(trimethylammonio)ethylphosphate, 2-((meth)acryloyloxy)hexyl-2'-(trimethylammonio)ethylphosphate, 2-(vinyloxy)ethyl-2'-(trimethylammonio)ethylphosphate, 2-(allyloxy)ethyl-2'-(trimethylammonio)ethylphosphate, 2-(p-vinylbenzyloxy)ethyl-2'-(trimethylammonio)ethylphosphate, 2-(p-vinylbenzoyloxy)ethyl-2'-(trimethylammonio)ethylphosphate, 2-(styryloxy)ethyl-2'-(trimethylammonio)ethylphosphate, 2-(p-vinylbenzyl)ethyl-2'-(trimethylammonio)ethylphosphate, 2-(vinyloxycarbonyl)ethyl-2'-(trimethylammonio)ethylphosphate, 2-(allyloxycarbonyl)ethyl-2'-(trimethylammonio)ethylphosphate, 2-(acryloylamino)ethyl-2'-(trimethylammonio)ethylphosphate, 2-(vinylcarbonylamino)ethyl-2'-(trimethylammonio)ethylphosphate, 2-(allyloxycarbonylamino)ethyl-2'-(trimethylammonio)ethylphosphate, 2-(butenoyloxy)ethyl-2'-(trimethylammonio)ethylphosphate, 2-(crotonoyloxy)ethyl-2'-(trimethylammonio)ethylphosphate, ethyl-(2'-trimethylammonio ethylphosphorylethyl)fumarate, butyl-(2'-trimethylammonio ethylphosphorylethyl)fumarate, hydroxyethyl-(2'-trimethylammonio ethylphosphorylethyl)fumarate, ethyl-(2'-trimethylammonio ethylphosphorylethyl)fumarate, butyl-(2'-trimethylammonio ethylphosphorylethyl)fumarate, and hydroxyethyl-(2'-trimethylammonio ethylphosphorylethyl)fumarate.

The average phosphorus (P) content of the surface-modified UV blocking powder may fall in the range of 0.01 ∼ 0.5 wt%, and the surface-modified UV blocking powder may be a UV blocking powder, a cosmetic powder or a coloring powder.

In addition, the present invention provides a UV blocking composition, a cosmetic composition or a coloring composition, comprising the surface-modified UV blocking powder as above.

### Advantageous Effects

According to the present invention, a surface-modified UV blocking powder can be prepared using an effective method, and is very stable by virtue of surface modification via chemical bonding (covalent bonding). Also, a UV blocking composition or the like containing the surface-modified UV blocking powder resulting from surface modification using a component having high biocompatibility can satisfy both UV blocking characteristics and biocompatibility.

### Mode for Invention

Hereinafter, a detailed description will be given of embodiments of the present invention. The following embodiments are set forth to more obviously illustrate, but are not to be construed as limiting the scope of the present invention.

As used herein, the term "chloroacetyl" refers to any material containing one to three chlorine atoms. Examples thereof include trichloroacetyl, dichloroacetyl and chloroacetyl.

According to an embodiment of the present invention, a surface-modified UV blocking powder is obtained by reacting a UV blocking powder having a chloroacetyl group, a phospholipid monomer having an unsaturated bond, and a catalyst for inducing the reaction of the chloroacetyl group and the unsaturated bond. When a UV blocking powder having UV blocking effectiveness is used as it is, it may cause skin trouble. Hence, it is surface-modified with a phospholipid monomer having superior biocompatibility. Surface modification is carried out through a chemical reaction in order to achieve superior stability and uniformity, compared to simple coating.

In order to react the UV blocking powder with the phospholipid monomer having an unsaturated bond such as MPC, account may be taken of a method of copolymerizing the UV blocking powder to which an unsaturated bond (a double bond) has been introduced, with a phospholipid monomer having an unsaturated bond using an initiator. However, this method is problematic because, in addition to the reaction between the unsaturated bond of the phospholipid monomer and the unsaturated bond of the UV blocking powder, the reaction of the unsaturated bond alone of the phospholipid monomer, regardless of the unsaturated bond of the UV blocking powder, occurs competitively. Hence, as the phospholipid monomer is required in an excessive amount, it is unnecessarily consumed. This method using a component material such as MPC, which is very expensive, is not considered to be a preferable surface modification method.

The present inventors have intensively studied the reaction starting from the unsaturated bond of the UV blocking powder to prevent unnecessary consumption of the phospholipid monomer such as MPC and thus have invented an effective surface modification method which allows the unsaturated bond of the UV blocking powder to react with the unsaturated bond of the phospholipid monomer while functioning as an initiator.

In an embodiment of the present invention, when the UV blocking powder is introduced with a chloroacetyl group and treated with a catalyst such as Mo(CO)₆, the -CCl₂· radical is produced, so that the initiation of the reaction occurs at the chloroacetyl group of the UV blocking powder, and the generated radical also functions as an initiator and thus reacts with the unsaturated bond of the phospholipid monomer. Such an addition reaction takes place once or several times, consequently forming an oligomer or a polymer.

The UV blocking powder is not limited so long as it has UV blocking effectiveness. The UV blocking powder may be an inorganic powder, and may include a composite comprising a variety of inorganic or organic-inorganic materials. For example, there is a composite powder comprising a core and one or two or more coating layers formed thereon. Also, it includes an inorganic powder in the composite form in which the core, as an organic material, is coated with an inorganic material, an inorganic powder coated with an organic material, or an inorganic powder partially containing an organic layer. Preferably useful is a powder having the -OH group on the surface thereof, and examples thereof may include titanium dioxide, zinc oxide, mica and talc. The average particle size of the UV blocking powder is not limited but may fall in the range of 50 ∼ 5000 nm.

The UV blocking powder having a chloroacetyl group may be represented by Chemical Formula 1 below.

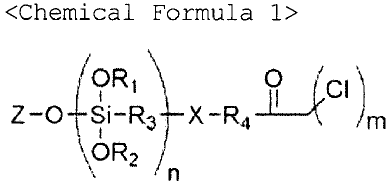

In Chemical Formula 1, Z is the aforementioned UV blocking powder. Z may contain no chloroacetyl or one or more chloroacetyl groups, and one or more chloroacetyl groups may be present in the structure as in Chemical Formula 1.

R₁ and R₂ are independently Z, hydrogen or a C1∼C20 aliphatic hydrocarbon with or without a substituent, and R₃ is oxygen or a C1∼C20 aliphatic hydrocarbon with or without a substituent and including a carbonyl group, a hydroxyl group, an ether group, an amine group, an amide group or a urethane group in a main chain or at a terminal thereof.

When both of R₁ and R₂ are Z, the adjacent three -O-groups present in a UV blocking powder may be linked with Si.

R₄ is absent (in this case, X is directly linked with the chloroacetyl group), or is a C1∼C20 aliphatic hydrocarbon with or without a substituent and including a carbonyl group, a hydroxyl group, an ether group, an amine group, an amide group or a urethane group in a main chain or at a terminal thereof.

X is absent, or is -OCONH-, -CONH-, -NH-, -CO-, -O-, -S-or a C1∼C20 aliphatic hydrocarbon with or without a substituent and including a carbonyl group, a hydroxyl group, an ether group, an amine group, an amide group or a urethane group in a main chain or at a terminal thereof.

n may be 0 or 1.

When n is 0, a chloroacetyl compound is directly reacted with the UV blocking powder. The chloroacetyl compound is not limited so long as it contains a chloroacetyl group and has a reactive group which may be linked with the UV blocking powder or the silane compound, and examples of the chloroacetyl compound may include trichloroacetyl isocyanate, dichloroacetyl isocyanate, chloroacetyl isocyanate and trichloroacetyl alkylisocyanate.

When n is 1, the silane compound plays a role as a linker. When the silane compound is provided as a linker in the middle portion of the compound, the reaction yield of the chloroacetyl compound may increase, compared to the direct reaction between the UV blocking powder and the chloroacetyl compound. Furthermore, various chloroacetyl compounds may be applied to the present invention. Examples of the silane compound may include, but are not limited to, tetraalkoxysilane, such as tetramethoxysilane, tetraethoxysilane and tetrapropylsilane, trialkoxyhydroxyalkylsilane, and trialkoxyaminoalkylamine.

m is an integer of 1 ∼ 3. Specifically, one, two or three chlorine atoms may be present.

Although not being limited, a preferred compound of Chemical Formula 1 may include the following examples, of which at least one compound may be used. All of the following compounds include three chlorine atoms, but the structures of compounds including one or two chlorine atoms will be sufficiently able to be understood.

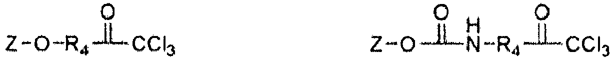

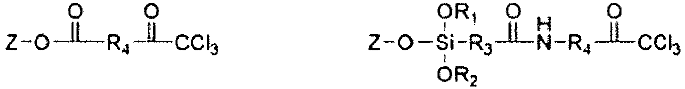

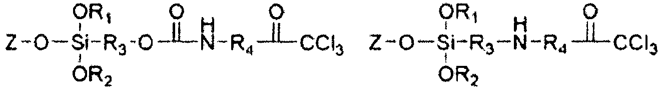

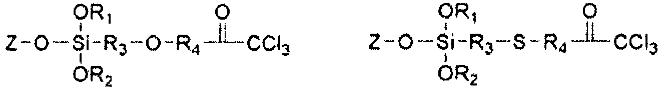

The phospholipid monomer having an unsaturated bond is not limited, but may be a phosphorylcholine analogous group-containing monomer having an unsaturated bond. It is preferably represented by Chemical Formula 2 below.

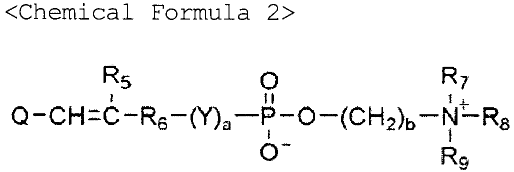

In Chemical Formula 2, R₆ is a divalent organic residue, Y is a C1∼C6 alkyleneoxy group, Q is a hydrogen atom or R₁₀-O-CO- (wherein R₁₀ is a C1∼C10 alkyl group or a C1∼C10 hydroxyalkyl group), R₅ is a hydrogen atom or a methyl group, R₇, R₈ and R₉ are each independently a hydrogen atom, a C1∼C6 hydrocarbon group or a hydroxyhydrocarbon group, a is 0 or 1, and b is an integer of 2 ∼ 4.

Specifically, the phospholipid monomer having an unsaturated bond may include at least one selected from among 2-((meth)acryloyloxy)ethyl-2'-(trimethylammonio)ethylphosphate, 3-((meth)acryloyloxy)propyl-2'-(trimethylammonio)ethylphosphate, 4-((meth)acryloyloxy)butyl-2'-(trimethylammonio)ethylphosphate, 5-((meth)acryloyloxy)pentyl-2'-(trimethylammonio)ethylphosphate, 6-((meth)acryloyloxy)hexyl-2'-(trimethylammonio)ethylphosphate, 2-((meth)acryloyloxy)ethyl-2'-(triethylammonio)ethylphosphate, 2-((meth)acryloyloxy)ethyl-2'-(tripropylammonio)ethylphosphate, 2-((meth)acryloyloxy)ethyl-2'-(tributylammonio)ethylphosphate, 2-((meth)acryloyloxy)propyl-2'-(trimethylammonio)ethylphosphate, 2-((meth)acryloyloxy)butyl-2'-(trimethylammonio)ethylphosphate, 2-((meth)acryloyloxy)pentyl-2'-(trimethylammonio)ethylphosphate, 2-((meth)acryloyloxy)hexyl-2'-(trimethylammonio)ethylphosphate, 2-(vinyloxy)ethyl-2'-(trimethylammonio)ethylphosphate, 2-(allyloxy)ethyl-2'-(trimethylammonio)ethylphosphate, 2-(p-vinylbenzyloxy)ethyl-2'-(trimethylammonio)ethylphosphate, 2-(p-vinylbenzoyloxy)ethyl-2'-(trimethylammonio)ethylphosphate, 2-(styryloxy)ethyl-2'-(trimethylammonio)ethylphosphate, 2-(p-vinylbenzyl)ethyl-2'-(trimethylammonio)ethylphosphate, 2-(vinyloxycarbonyl)ethyl-2'-(trimethylammonio)ethylphosphate, 2-(allyloxycarbonyl)ethyl-2'-(trimethylammonio)ethylphosphate, 2-(acryloylamino)ethyl-2'-(trimethylammonio)ethylphosphate, 2-(vinylcarbonylamino)ethyl-2'-(trimethylammonio)ethylphosphate, 2-(allyloxycarbonylamino)ethyl-2'-(trimethylammonio)ethylphosphate, 2-(butenoyloxy)ethyl-2'-(trimethylammonio)ethylphosphate, 2-(crotonoyloxy)ethyl-2'-(trimethylammonio)ethylphosphate, ethyl-(2'-trimethylammonio ethylphosphorylethyl)fumarate, butyl-(2'-trimethylammonio ethylphosphorylethyl)fumarate, hydroxyethyl-(2'-trimethylammonio ethylphosphorylethyl)fumarate, ethyl-(2'-trimethylammonio ethylphosphorylethyl)fumarate, butyl-(2'-trimethylammonio ethylphosphorylethyl)fumarate, and hydroxyethyl-(2'-trimethylammonioethylphosphorylethyl)fumarate.

Among the phosphorylcholine analogous group-containing compounds, the term "(meth)acryl" refers to both acryl and methacryl.

The catalyst which induces the reaction between the chloroacetyl group and the unsaturated bond is not limited so long as it produces a radical on the chloroacetyl group, and is exemplified by Mo(CO)₆.

Meanwhile, copolymerization may be implemented with the additional use of a radical polymerizable monomer. The monomer for copolymerization preferably includes an α,β-ethylenically unsaturated compound having an ethylene bond that is an unsaturated double bond between carbon atoms at Positions α and β, for example, α,β-ethylenically unsaturated carboxylic acid and a nonionic α,β-ethylenically unsaturated material. Examples of the α,β-ethylenically unsaturated carboxylic acid may include monobasic acid such as acrylic acid, methacrylic acid, crotonic acid and acyloxypropionic acid, and dibasic acid such as maleic acid, fumaric acid and itaconic acid, which may be used alone or in combination. Examples of the nonionic α,β-ethylenically unsaturated material may include methyl acrylate, ethyl acrylate, propyl acrylate, butyl acrylate, 2-ethyl hexyl acrylate, methyl methacrylate, ethyl methacrylate, butyl methacrylate, 2-hydroxybutyl acrylate, 2-hydroxyethyl methacrylate, 2-hydroxypropyl methacrylate, styrene, butadiene, isoprene, vinyl chloride, vinylidene chloride, acrylonitrile, methacrylonitrile, vinyl acetate, vinyl butyrate, vinyl caproate, p-chlorostyrene, isopropyl styrene and vinyl toluene, which may be alone or in combination.

When the radical polymerizable monomer is used together for copolymerization, the amount thereof is not limited but may be set to the range of 0.5 ∼ 99.5 mol per 100 mol of MPC. Preferably, it is used in an amount of 20 ∼ 90 mol. It may exhibit a desired copolymerization effect when used in the amount of 20 mol or more. If the amount thereof exceeds 90 mol, usability of the resulting formulation may become poor.

The average phosphorus (P) content of the surface-modified UV blocking powder may fall in the range of 0.01 ∼ 0.5 wt%. If the P content is less than the above lower limit, particle dispersibility may decrease and there is thus no improvement in UV blocking performance. In contrast, if it exceeds the above upper limit, the resulting formulation may become sticky.

The surface-modified UV blocking powder may be used in an amount of 0.1 ∼ 10 wt% based on the amount of MPC.

In addition, the present invention provides a UV blocking composition, a coloring composition or a cosmetic composition, comprising the surface-modified UV blocking powder as above. In the present invention, the UV blocking composition is mainly employed to block UV rays, and the coloring composition is used to exhibit a specific color and may further manifest UV blocking effectiveness. The cosmetic composition is applied to the human body so that the human body is made clean and beautiful to thus obtain an attractive and bright appearance or in order to keep or enhance the health of the skin or hair, and is a broad concept including personal hygiene items such as detergents and bath solvents.

The amount of the surface-modified UV blocking powder in the UV blocking composition, the coloring composition or the cosmetic composition may vary depending on the end use and the formulation, and is preferably set to 1 ∼ 40 wt% based on the total weight of the composition in terms of usability (e.g. spreadability) and moisture retention.

The aforementioned composition according to the present invention may further include an aqueous medium, and an oil medium, such as a higher fatty acid, higher alcohol or hydrocarbon, in addition to the surface-modified UV blocking powder. Also, it may be appropriately mixed with ester, silicone, an anionic surfactant, a cationic surfactant, an amphoteric surfactant, a nonionic surfactant, a water-soluble polymer, a film coating agent, a UV absorber, a sequestering agent, lower alcohol, polyhydric alcohol, sugar, amino acid, organic amine, a polymer emulsion, a pH controller, a skin nutrient, vitamin, an antioxidant, an antioxidant aid, or a fragrance, as necessary. The resulting composition may be prepared into a variety of desired formulations including toner, essence, lotion and wax, using a known method.

A better understanding of the present invention may be obtained through the following examples which are set forth to more clearly explain, but are not to be construed to limit the scope of the present invention.

### <Example 1>

### (1) Preparation of UV blocking powder having trichloroacetyl group

20 g of titanium dioxide (after drying), 600 g of toluene, and 2.0 g of trichloroacetyl isocyanate were placed in a 1 L flask, stirred under reflux at 50 ∼ 100°C for 5 ∼ 10 hr, and then cooled to room temperature. The mixture was centrifuged to remove the supernatant (the solvent layer), dispersed in THF and then centrifuged again. These procedures were repeated until the unreacted trichloroacetyl isocyanate was completely removed, followed by drying in a vacuum to remove the residual THF solvent and then drying in a vacuum, thus obtaining a UV blocking powder having a trichloroacetyl group.

### (2) Preparation of surface-modified UV blocking powder

20 g of the UV blocking powder having a trichloroacetyl group, 4 g of MPC (Methacryloyloxyethyl phosphorylcholine), 0.04 g of Mo(CO)₆, and 200 g of ethanol were placed in a 500 L flask, and maintained at 50 ∼ 90°C for 1 ∼ 4 hr and thus polymerized. Cooling to room temperature and centrifugation to remove the supernatant (the solvent layer) were carried out, after which ethanol was added, and the resulting mixture was stirred, cleaned and centrifuged, thus separating and removing the supernatant. The resulting particle was dried in a vacuum and residual ethanol was thus removed, thereby obtaining a surface-modified UV blocking powder comprising titanium dioxide coated with MPC.

This powder was analyzed by XRF and ICP and was confirmed to be coated with 5.0% of MPC.

### <Example 2>

### (1) Preparation of UV blocking powder having trichloroacetyl group

The present example was carried out in the same manner as in Example 1, with the exception that, before reaction between titanium dioxide (after drying) and trichloroacetyl isocyanate, titanium dioxide was reacted with tetraethoxysilane (TEOS) and then with trichloroacetyl isocyanate.

20 g of titanium dioxide (after drying), 600 g of toluene, and 30 g of tetraethoxysilane (TEOS) were placed in a 1 L flask, stirred under reflux at 70 ∼ 120°C for 5 ∼ 10 hr and then cooled to room temperature. Subsequently, centrifugation to remove the supernatant (the solvent layer), dispersion in THF, centrifugation and drying in a vacuum were performed, thus obtaining a powder comprising titanium dioxide and tetraethoxysilane.

20 g of the powder comprising titanium dioxide and tetraethoxysilane, 200 g of methanol, and 20 g of 0.01M HCl were placed in a 1 L flask, stirred at 20 ∼ 70°C for 30 min ∼ 1 hr, centrifuged to remove the supernatant (the solvent layer), cleaned with methanol, centrifuged to remove the solvent layer, and dried in a vacuum, thus removing residual methanol.

20 g of the obtained UV blocking powder, 600 g of toluene, and 2.0 g of trichloroacetyl isocyanate were placed in a 1 L flask, stirred under reflux at 50 ∼ 100°C for 5 ∼ 10 hr and then cooled to room temperature. Centrifugation to remove the supernatant (the solvent layer), dispersion in THF and then centrifugation were performed. These procedures were repeated until the unreacted trichloroacetyl isocyanate was completely removed, followed by drying in a vacuum to remove the residual THF solvent and then further drying in a vacuum, thus obtaining a UV blocking powder having a trichloroacetyl group.

### (2) Preparation of surface-modified UV blocking powder

20 g of the UV blocking powder having a trichloroacetyl group, 4 g of MPC (Methacryloyloxyethyl phosphorylcholine), 0.04 g of Mo(CO)₆, and 200 g of ethanol were placed in a 500 L flask, and maintained at 50 ∼ 90°C for 1 ∼ 4 hr and thus polymerized. Subsequently, cooling to room temperature, centrifugation to remove the supernatant (the solvent layer), addition of ethanol, stirring, cleaning and centrifugation to remove the supernatant were performed. The resulting particle was dried in a vacuum and residual ethanol was thus removed, thereby obtaining a surface-modified UV blocking powder comprising titanium dioxide coated with MPC.

This powder was analyzed by XRF and ICP and was confirmed to be coated with 4.0% of MPC.

### <Example 3>

### (1) Preparation of UV blocking powder having chloroacetyl group

20 g of titanium dioxide (after drying), 600 g of toluene, and 2.0 g of chloroacetyl isocyanate were placed in a 1 L flask, stirred under reflux at 50 ∼ 100°C for 5 ∼ 10 hr and then cooled to room temperature. Subsequently, centrifugation to remove the supernatant (the solvent layer), dispersion in THF and centrifugation were performed. These procedures were repeated until the unreacted chloroacetyl isocyanate was completely removed, followed by drying in a vacuum to remove the residual THF solvent and then further drying in a vacuum, thus obtaining a UV blocking powder having a trichloroacetyl group.

### (2) Preparation of surface-modified UV blocking powder

20 g of the UV blocking powder having a chloroacetyl group, 4 g of MPC (Methacryloyloxyethyl phosphorylcholine), 0.04 g of Mo(CO)₆, and 200 g of ethanol were placed in a 500 L flask, and maintained at 50 ∼ 90°C for 1 ∼ 4 hr and thus polymerized. Subsequently, cooling to room temperature, centrifugation to remove the supernatant (the solvent layer), addition of ethanol, stirring, cleaning and centrifugation to separate and remove the supernatant were performed. The resulting particle was dried in a vacuum and residual ethanol was thus removed, thereby obtaining a surface-modified UV blocking powder comprising titanium dioxide coated with MPC.

This powder was analyzed by XRF and ICP and was confirmed to be coated with 1.0% of MPC.

### <Example 4>

### (1) Preparation of UV blocking powder having trichloroacetyl group

20 g of zinc oxide (after drying), 600 g of toluene, and 2.0 g of trichloroacetyl isocyanate were placed in a 1 L flask, stirred under reflux at 50 ∼ 100°C for 5 ∼ 10 hr and then cooled to room temperature. Subsequently, centrifugation to remove the supernatant (the solvent layer), dispersion in THF and centrifugation were performed. These procedures were repeated until the unreacted trichloroacetyl isocyanate was completely removed, followed by drying in a vacuum to remove the residual THF solvent and then further drying in a vacuum, thus obtaining a UV blocking powder having a trichloroacetyl group.

### (2) Preparation of surface-modified UV blocking powder

20 g of the UV blocking powder having a trichloroacetyl group, 4 g of MPC (Methacryloyloxyethyl phosphorylcholine), 0.04 g of Mo(CO)₆, and 200 g of ethanol were placed in a 500 L flask, and maintained at 50 ∼ 90°C for 1 ∼ 4 hr and thus polymerized. Subsequently, cooling to room temperature, centrifugation to remove the supernatant (the solvent layer), addition of ethanol, stirring, cleaning and centrifugation to separate and remove the supernatant were carried out. The resulting particle was dried in a vacuum and residual ethanol was thus removed, thereby obtaining a surface-modified UV blocking powder comprising zinc oxide coated with MPC.

This powder was analyzed by XRF and ICP and was confirmed to be coated with 5.0% of MPC.

### <Example 5>

### (1) Preparation of UV blocking powder having trichloroacetyl group

20 g of titanium dioxide (after drying), 600 g of toluene, and 30 g of tetramethoxysilane (TMOS) were placed in a 1 L flask, stirred under reflux at 70 ∼ 120°C for 5 ∼ 10 hr and then cooled to room temperature. Subsequently, centrifugation to remove the supernatant (the solvent layer), dispersion in THF, centrifugation and drying in a vacuum were conducted, thus obtaining a powder comprising titanium dioxide and tetramethoxysilane.

20 g of the powder comprising titanium dioxide and tetramethoxysilane, 200 g of methanol, and 20 g of 0.01M HCl were placed in a 1 L flask, stirred at 20 ∼ 70°C for 30 min ∼ 1 hr, centrifuged to remove the supernatant (the solvent layer), cleaned with methanol, centrifuged to remove the solvent layer, and dried in a vacuum, thus removing residual methanol.

20 g of the obtained UV blocking powder, 600 g of toluene, and 2.0 g of trichloroacetyl isocyanate were placed in a 1 L flask, stirred under reflux at 50 ∼ 100°C for 5 ∼ 10 hr, and cooled to room temperature. Subsequently, centrifugation to remove the supernatant (the solvent layer), dispersion in THF and centrifugation were performed. These procedures were repeated until the unreacted trichloroacetyl isocyanate was completely removed, followed by drying in a vacuum to remove the residual THF solvent and then further drying in a vacuum, thus obtaining a UV blocking powder having a trichloroacetyl group.

### (2) Preparation of surface-modified UV blocking powder

20 g of the UV blocking powder having a trichloroacetyl group, 4 g of MPC (Methacryloyloxyethyl phosphorylcholine), 0.04 g of Mo(CO)₆, and 200 g of ethanol were placed in a 500 L flask, and maintained at 50 ∼ 90°C for 1 ∼ 4 hr and thus polymerized. Subsequently, cooling to room temperature, centrifugation to remove the supernatant (the solvent layer), addition of ethanol, stirring, cleaning and centrifugation to separate and remove the supernatant were implemented. The resulting particle was dried in a vacuum and residual ethanol was thus removed, thereby obtaining a surface-modified UV blocking powder comprising titanium dioxide coated with MPC.

This powder was analyzed by XRF and ICP and was confirmed to be coated with 5.0% of MPC.

### Preparative Examples 1 to 5 and Comparative Example 1

In Preparative Examples 1 to 5, sun creams were prepared using, as a UV blocking powder, the surface-modified UV blocking powders of Examples 1 to 5 and components as shown in Table 1 below. Also, in Comparative Example 1, a sun cream was prepared using, as a UV blocking powder, a titanium dioxide powder which was not surface-modified.

**[Table 1]**

| Component | Prep.Ex.1 | Prep.Ex.2 | Prep.Ex.3 | Prep.Ex.4 | Prep.Ex.5 | Comp.Ex.1 |
|---|---|---|---|---|---|---|
| Distilled water | 65 | 65 | 65 | 65 | 65 | 65 |
| Dissolvine Na2-S | 0.1 | 0.1 | 0.1 | 0.1 | 0.1 | 0.1 |
| Propylene Glycol | 2.0 | 2.0 | 2.0 | 2.0 | 2.0 | 2.0 |
| Finsolv TN | 3.0 | 3.0 | 3.0 | 3.0 | 3.0 | 3.0 |
| Protachem SMO | 0.2 | 0.2 | 0.2 | 0.2 | 0.2 | 0.2 |
| Escalol 557 Octinoxate | 7.5 | 7.5 | 7.5 | 7.5 | 7.5 | 7.5 |
| Escalol | 3.0 | 3.0 | 3.0 | 3.0 | 3.0 | 3.0 |
| 567 Oxybenzone | | | | | | |
| Escalol 587 | 3.0 | 3.0 | 3.0 | 3.0 | 3.0 | 3.0 |
| Crodafos CES | 4.0 | 4.0 | 4.0 | 4.0 | 4.0 | 4.0 |
| Ceterayl Alcohol 50/50 | 4.0 | 4.0 | 4.0 | 4.0 | 4.0 | 4.0 |
| Crodafos C320 Acid | 1.3 | 1.3 | 1.3 | 1.3 | 1.3 | 1.3 |
| Liquid Germall Plus | 0.6 | 0.6 | 0.6 | 0.6 | 0.6 | 0.6 |
| Fragrance | 0.2 | 0.2 | 0.2 | 0.2 | 0.2 | 0.2 |
| TEA 99% | 0.1 | 0.1 | 0.1 | 0.1 | 0.1 | 0.1 |
| UV Blocking Powder | 6.0 Ex.1 | 6.0 Ex.2 | 6.0 Ex.3 | 6.0 Ex.4 | 6.0 Ex.5 | 6.0 |

| | | | | | | |
|---|---|---|---|---|---|---|
| **all of the units of components are "g." | | | | | | |

### <Test Example>

### (1) Applicability (Spreadability)

The applicability (spreadability) was evaluated through panel testing when the sun creams of Preparative Examples 1 to 5 and Comparative Example 1 were applied on subject faces. The results are given in Table 2 below. Ultimately, the applicability (spreadability) was superior.

**[Table 2]**

| Panel | Prep.Ex.1 | Prep.Ex.2 | Prep.Ex.3 | Prep.Ex.4 | Prep.Ex.5 | Comp.Ex.1 |
|---|---|---|---|---|---|---|
| PJY | 10 | 8 | 10 | 10 | 10 | 5 |
| MJL | 10 | 10 | 8 | 5 | 10 | 3 |
| LSS | 10 | 10 | 5 | 10 | 5 | 1 |
| LMI | 10 | 10 | 5 | 8 | 10 | 5 |
| SSM | 10 | 10 | 5 | 10 | 8 | 5 |
| PYS | 10 | 10 | 8 | 10 | 10 | 3 |
| HYA | 10 | 10 | 8 | 10 | 10 | 5 |
| KJH | 10 | 8 | 8 | 5 | 10 | 3 |
| YSS | 10 | 10 | 5 | 10 | 5 | 3 |
| KJK | 10 | 10 | 8 | 5 | 5 | 1 |

| | | | | | | |
|---|---|---|---|---|---|---|
| (10: very good/ 8: good/ 5: fair/ 3: poor/ 1: very poor) | | | | | | |

### (2) Adhesion

The adhesion was evaluated through panel testing when the sun creams of Preparative Examples 1 to 5 and Comparative Example 1 were applied on subject faces. The results are given in Table 3 below. Ultimately, the adhesion was superior.

**[Table 3]**

| Panel | Prep.Ex.1 | Prep.Ex.2 | Prep.Ex.3 | Prep.Ex.4 | Prep.Ex.5 | Comp.Ex.1 |
|---|---|---|---|---|---|---|
| PJY | 10 | 10 | 8 | 10 | 10 | 3 |
| MJL | 10 | 8 | 8 | 3 | 8 | 3 |
| LSS | 9 | 10 | 5 | 5 | 10 | 1 |
| LMI | 10 | 10 | 5 | 8 | 3 | 3 |
| SSM | 10 | 10 | 5 | 10 | 10 | 5 |
| PYS | 10 | 10 | 8 | 8 | 8 | 1 |
| HYA | 8 | 10 | 5 | 8 | 10 | 3 |
| KJH | 10 | 10 | 8 | 10 | 5 | 3 |
| YSS | 10 | 10 | 10 | 5 | 5 | 3 |
| KJK | 10 | 10 | 8 | 5 | 5 | 1 |

| | | | | | | |
|---|---|---|---|---|---|---|
| (10: very good/ 8: good/ 5: fair/ 3: poor/ 1: very poor) | | | | | | |

### (3) SPF (in vitro)

SPF (Sun Protection Factor) (in vitro) of the sun creams prepared in Preparative Examples 1 to 5 and Comparative Example 1 was measured by a typical method using a SPF-290S analyzer. The results are given in Table 4 below. Based on the SPF measurement results, SPF of the sun creams (powders coated with MPC polymer) of Preparative Examples 1 to 5 according to the present invention was increased by approximately 15 ∼ 30% compared to Comparative Example 1 (non-coated powder).

**[Table 4]**

| | Prep.Ex.1 | Prep.Ex.2 | Prep.Ex.3 | Prep.Ex.4 | Prep.Ex.5 | Comp.Ex.1 |
|---|---|---|---|---|---|---|
| SPF | 39.0 | 36.0 | 34.0 | 38.0 | 37.4 | 29.0 |

When a UV blocking composition is prepared using the UV blocking powder coated with the MPC polymer according to the present invention, superior spreadability and adhesion can be exhibited compared to when using the UV blocking powder which is not coated with the MPC polymer. Also, SPF is increased by approximately 15 ∼ 30%. Therefore, the surface-modified UV blocking powder according to the present invention can yield excellent characteristics when used in cosmetics.

### Industrial Applicability

According to the present invention, a surface-modified UV blocking powder can be prepared using an effective method, and is very stable by virtue of surface modification via chemical bonding (covalent bonding). Also, a UV blocking composition or the like containing the surface-modified UV blocking powder resulting from surface modification using a component having high biocompatibility can satisfy both UV blocking characteristics and biocompatibility and is thus industrially useful.

## Claims

1. A surface-modified ultraviolet blocking powder, obtained by reacting an ultraviolet blocking powder having a chloroacetyl group, a phospholipid monomer having an unsaturated bond, and a catalyst for inducing a reaction of the chloroacetyl group and the unsaturated bond.

2. The surface-modified ultraviolet blocking powder of claim 1, wherein the ultraviolet blocking powder having a chloroacetyl group is represented by Chemical Formula 1 below. 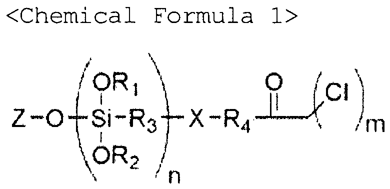 (in Chemical Formula 1, Z is an ultraviolet blocking powder, R₁ and R₂ are independently Z, hydrogen or a C1∼C20 aliphatic hydrocarbon with or without a substituent, R₃ is oxygen or a C1∼C20 aliphatic hydrocarbon with or without a substituent and including a carbonyl group, a hydroxyl group, an ether group, an amine group, an amide group or a urethane group in a main chain or at a terminal thereof, R₄ is absent or is a C1∼C20 aliphatic hydrocarbon with or without a substituent and including a carbonyl group, a hydroxyl group, an ether group, an amine group, an amide group or a urethane group in a main chain or at a terminal thereof, X is absent or is -OCONH-, -CONH-, -NH-, -CO-, -O-, -S- or a C1∼C20 aliphatic hydrocarbon with or without a substituent and including a carbonyl group, a hydroxyl group, an ether group, an amine group, an amide group or a urethane group in a main chain or at a terminal thereof, n is 0 or 1, and m is an integer of 1 ∼ 3.)

3. The surface-modified ultraviolet blocking powder of claim 1, wherein the ultraviolet blocking powder comprises titanium dioxide, zinc oxide, mica, sericite or talc.

4. The surface-modified ultraviolet blocking powder of claim 1, wherein the catalyst is Mo(CO)₆.

5. The surface-modified ultraviolet blocking powder of claim 2, wherein a compound of Chemical Formula 1 comprises at least one selected from among compounds of the following Chemical Formulas.

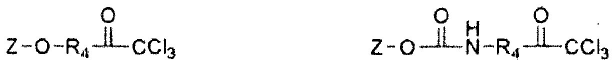

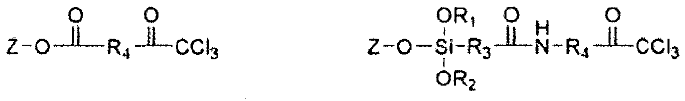

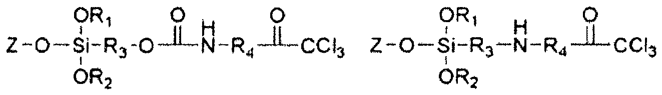

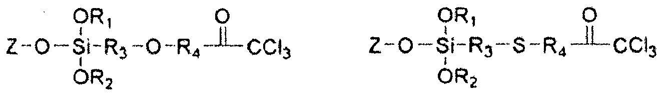

6. The surface-modified ultraviolet blocking powder of claim 2, wherein in Chemical Formula 1, when either or both of R₁ and R₂ are Z, all Zs represented in the Chemical Formula are the same ultraviolet blocking powder.

7. The surface-modified ultraviolet blocking powder of claim 1, wherein the phospholipid monomer having an unsaturated bond is a phosphorylcholine analogous group-containing monomer having an unsaturated bond.

8. The surface-modified ultraviolet blocking powder of claim 1, wherein the phospholipid monomer having an unsaturated bond is a phosphorylcholine analogous group-containing monomer represented by Chemical Formula 2 below. 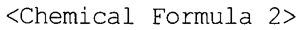 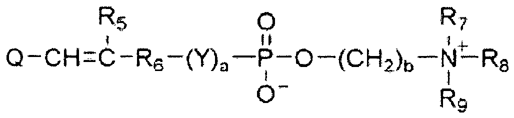 (in Chemical Formula 2, R₆ is a divalent organic residue, Y is a C1∼C6 alkyleneoxy group, Q is a hydrogen atom or R₁₀-O-CO- (wherein R₁₀ is a C1∼C10 alkyl group or a C1∼C10 hydroxyalkyl group), R₅ is a hydrogen atom or a methyl group, R₇, R₈ and R₉ are each independently a hydrogen atom, a C1∼C6 hydrocarbon group or a hydroxyhydrocarbon group, a is 0 or 1, and b is an integer of 2 ∼ 4.)

9. The surface-modified ultraviolet blocking powder of claim 1, wherein the phospholipid monomer having an unsaturated bond comprises at least one selected from among:
2-(((meth)acryloyloxy)ethyl-2'-(trimethylammonio)ethylphosphate,
3-(((meth)acryloyloxy)propyl-2'-(trimethylammonio)ethylphosphate,
4-(((meth)acryloyloxy)butyl-2'-(trimethylammonio)ethylphosphate,
5-(((meth)acryloyloxy)pentyl-2'-(trimethylammonio)ethylphosphate,
6-(((meth)acryloyloxy)hexyl-2'-(trimethylammonio)ethylphosphate,
2-(((meth)acryloyloxy)ethyl-2'-(triethylammonio)ethylphosphate,
2-(((meth)acryloyloxy)ethyl-2'-(tripropylammonio)ethylphosphate,
2-(((meth)acryloyloxy)ethyl-2'-(tributylammonio)ethylphosphate,
2-(((meth)acryloyloxy)propyl-2'-(trimethylammonio)ethylphosphate,
2-(((meth)acryloyloxy)butyl-2'-(trimethylammonio)ethylphosphate,
2-(((meth)acryloyloxy)pentyl-2'-(trimethylammonio)ethylphosphate,
2-(((meth)acryloyloxy)hexyl-2'-(trimethylammonio)ethylphosphate,
2-((vinyloxy)ethyl-2'-(trimethylammonio)ethylphosphate,
2-((allyloxy)ethyl-2'-(trimethylammonio)ethylphosphate,
2-((p-vinylbenzyloxy)ethyl-2'-(trimethylammonio)ethylphosphate,
2-((p-vinylbenzoyloxy)ethyl-2'-(trimethylammonio)ethylphosphate,
2-((styryloxy)ethyl-2'-(trimethylammonio)ethylphosphate,
2-((p-vinylbenzyl)ethyl-2'-(trimethylammonio)ethylphosphate,
2-((vinyloxycarbonyl)ethyl-2'-(trimethylammonio)ethylphosphate,
2-((allyloxycarbonyl)ethyl-2'-(trimethylammonio)ethylphosphate,
2-((acryloylamino)ethyl-2'-(trimethylammonio)ethylphosphate,
2-((vinylcarbonylamino)ethyl-2'-(trimethylammonio)ethylphosphate,
2-((allyloxycarbonylamino)ethyl-2'-(trimethylammonio)ethylphosphate,
2-((butenoyloxy)ethyl-2'-(trimethylammonio)ethylphosphate,
2-((crotonoyloxy)ethyl-2'-(trimethylammonio)ethylphosphate,
ethyl-(2'-trimethylammonio ethylphosphorylethyl)fumarate,
butyl-(2'-trimethylammonio ethylphosphorylethyl)fumarate,
hydroxyethyl-(2'-trimethylammonio ethylphosphorylethyl)fumarate,
ethyl-(2'-trimethylammonio ethylphosphorylethyl)fumarate,
butyl-(2'-trimethylammonio ethylphosphorylethyl)fumarate, and
hydroxyethyl-(2'-trimethylammonio ethylphosphorylethyl)fumarate.

10. The surface-modified ultraviolet blocking powder of claim 2, wherein an average phosphorus (P) content of the surface-modified ultraviolet blocking powder falls in a range of 0.01 ∼ 0.5 wt%.

11. The surface-modified ultraviolet blocking powder of claim 1, wherein the surface-modified ultraviolet blocking powder is an ultraviolet blocking powder, a cosmetic powder or a coloring powder.

12. An ultraviolet blocking composition, comprising the surface-modified ultraviolet blocking powder of any one of claims 1 to 11.

13. A cosmetic composition, comprising the surface-modified ultraviolet blocking powder of any one of claims 1 to 11.

14. A coloring composition, comprising the surface-modified ultraviolet blocking powder of any one of claims 1 to 11.
